(19) Europäisches Patentamt
European Patent Office
Office européen des brevets

(11) **EP 3 071 292 B1**

(12) **FASCICULE DE BREVET EUROPEEN**

(45) Date de publication et mention de la délivrance du brevet:
**13.03.2019 Bulletin 2019/11**

(21) Numéro de dépôt: **14821721.9**

(22) Date de dépôt: **20.11.2014**

(51) Int Cl.:
***A61N 5/10*** (2006.01)

(86) Numéro de dépôt international:
**PCT/FR2014/052979**

(87) Numéro de publication internationale:
**WO 2015/075388 (28.05.2015 Gazette 2015/21)**

(54) **DISPOSITIF D'IRRADIATION A RAYONNEMENT IONISANT NOTAMMENT POUR LA RADIOTHÉRAPIE ET/OU LA RADIOBIOLOGIE**

BESTRAHLUNGSVORRICHTUNG MIT IONISIERENDER STRAHLUNG, INSBESONDERE FÜR RADIOTHERAPIE UND/ODER RADIOBIOLOGIE

IRRADIATION DEVICE USING IONIZING RADIATION, PARTICULARLY FOR RADIOTHERAPY AND/OR RADIOBIOLOGY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.11.2013 FR 1302672**

(43) Date de publication de la demande:
**28.09.2016 Bulletin 2016/39**

(73) Titulaire: **P M B**
**13790 Peynier (FR)**

(72) Inventeur: **LIGER, Philippe**
**13790 Peynier (FR)**

(74) Mandataire: **Santarelli**
**49, avenue des Champs-Elysées**
**75008 Paris (FR)**

(56) Documents cités:
**WO-A2-2012/085507**

- **HAFIZ M ZIN ET AL: "Paper;Towards real-time VMAT verification using a prototype, high-speed CMOS active pixel sensor;Towards real-time VMAT verification using a prototype, high-speed CMOS active pixel sensor", PHYSICS IN MEDICINE AND BIOLOGY, INSTITUTE OF PHYSICS PUBLISHING, BRISTOL GB, vol. 58, no. 10, 25 avril 2013 (2013-04-25), pages 3359-3375, XP020244770, ISSN: 0031-9155, DOI: 10.1088/0031-9155/58/10/3359**
- **LI JI ET AL: "Improvements in dose accuracy delivered with static-MLC IMRT on an integrated linear accelerator control system", MEDICAL PHYSICS, AIP, MELVILLE, NY, US, vol. 39, no. 5, 1 mai 2012 (2012-05-01), pages 2456-2462, XP012161002, ISSN: 0094-2405, DOI: 10.1118/1.3701778 [extrait le 2012-04-13]**

## Description

La présente invention concerne un dispositif d'irradiation à rayonnement ionisant notamment pour la radiothérapie et/ou la radiologie capable de délivrer de manière contrôlée et précise, de façon programmée, de fortes doses de rayonnement ionisant d'au moins 0,25 Gy, de préférence 10 Gy, avec une précision d'au moins 1 $\mu$Gy, de préférence 1 nGy, dans une gamme d'énergie comprise entre 1 MeV et 50 MeV, en des instants très brefs, c'est-à-dire par exemple d'au moins 0,1 $\mu$s, de préférence 100 $\mu$s, voire 1ms, ou encore 100 ms.

Elle concerne également un dispositif d'irradiation à rayonnement ionisant doté d'un système de contrôle d'impulsion de puissance capable de produire un faisceau de particules d'énergie ajustable dans une gamme comprise entre 1 MeV et 50 MeV, pulsé à une fréquence (f) souhaitée avec une durée d'impulsion (d) ajustable d'au moins 1 ns, de préférence 0,1 $\mu$s, et capable de délivrer un débit de dose absorbée jusqu'à 250 Gy/s voire jusqu'à 500 Gy/s ou même encore jusqu'à 1000 Gy/s.

Elle concerne plus particulièrement un dispositif d'irradiation à rayonnement ionisant notamment pour la radiothérapie et/ou la radiobiologie dans lequel les différents moyens et systèmes le constituant sont interconnectés entre eux de façon intelligente pour coopérer et former une boucle de contrôle et régulation, notamment de la puissance, de façon à délivrer de manière contrôlée et précise de fortes doses de rayonnement ionisant d'au moins 0,25 Gy avec une précision d'au moins 1 $\mu$Gy, de préférence 1 nGy, à des énergies dans une gamme comprise entre 1 MeV et 50 MeV, pendant des instants très brefs, c'est-à-dire par exemple d'au moins 0,1 $\mu$s, de préférence 100 $\mu$s, voire 1 ms, ou encore 100 ms.

Elle concerne éventuellement un dispositif d'irradiation à rayonnement ionisant notamment pour la radiothérapie et/ou la radiobiologie comprenant un détecteur rapide, également qualifié « ultra-rapide », capable de détecter une dose en des temps très courts, par exemple en au moins 0,01 ns, couplé à une électronique de contrôle capable de contrôler une dose délivrée pendant une fraction de seconde, par exemple pendant au moins 0,1$\mu$s, de préférence 1 $\mu$s ou 1 ms, et de préférence pendant moins d'une seconde, voire moins de 200 ms.

La radiothérapie est une méthode de traitement locorégional des cancers. Elle est avec la chirurgie, le traitement le plus fréquent des cancers et peut entrainer une rémission nette à elle seule. Elle peut être utilisée seule ou associée à la chirurgie et à la chimiothérapie. Ses indications sont liées au type de tumeur, à sa localisation, à son stade et à l'état général de la cible. Elle présente dans certains cas, l'avantage d'être réalisée en ambulatoire par le fait que les séances peuvent être de courtes durées et les effets secondaires moindres que ceux d'une chimiothérapie. Pour ce faire, la radiothérapie utilise des radiations pour détruire les cellules cancéreuses en affectant leur capacité à se reproduire. L'irradiation a pour but de détruire toutes les cellules tumorales tout en épargnant les tissus sains périphériques.

Le document WO 2012/085507 décrit par exemple un procédé, et un dispositif associé, pour contrôler spatialement l'incidence d'un faisceau irradiant. En effet, pour certains types de cancer un traitement par rayons X présente la difficulté que la tumeur peut être localisée très près de partie du coeur qu'il peut être préférable d'éviter d'irradier.

De façon générale, un dispositif d'irradiation à rayonnement ionisant pour la radiothérapie et/ou la radiobiologie comprend un accélérateur linéaire de faisceau d'électrons ou d'ions et une électronique de contrôle et de commande qui permet d'arrêter globalement l'émission de rayonnement ionisant lorsque la dose prescrite par l'opérateur est atteinte.

Les machines de radiothérapie conventionnelles permettent de délivrer à ce jour, du rayonnement ionisant sous forme d'électrons ou de rayons X à des énergies de 3 à 25 MeV, à des doses de l'ordre de 1 Gy, avec un débit de dose de l'ordre de 4 Gy par minute et avec une précision médiocre.

Des solutions ont été proposées pour obtenir une précision des doses de rayonnement délivrées de l'ordre de 1%. En effet, les temps d'irradiation, les doses délivrées et les collimations sont désormais programmés par un opérateur physicien médical en coopération avec un radiothérapeute à l'aide d'outils informatiques. Malheureusement, cette précision ne peut pas être atteinte avec les dispositifs conventionnels de détection et de contrôle de la dose et/ou du débit de dose délivrée et/ou absorbée. En effet, les problèmes majeurs de ces dispositifs se caractérisent par le fait qu'ils ne sont pas capables de délivrer de manière contrôlée et précise, de façon programmée et intelligente, de fortes doses de rayonnement ionisant jusqu'à 250 Gy/s, voire jusqu'à 500 Gy/s ou même encore jusqu'à 1000 Gy/s, à des énergies dans une gamme comprise entre 1 MeV et 50 MeV, en des instants très brefs, c'est-à-dire par exemple d'au moins 0,1 $\mu$s, de préférence 100 $\mu$s, voire 1 ms ou encore 100 ms.

Un autre problème technique majeur de ces dispositifs est que les différents organes composant lesdits dispositifs ne sont pas suffisamment rapides et/ou interconnectés entre eux de façon intelligente pour coopérer ensemble et former une boucle suffisamment rapide de régulation et de contrôle de la dose et/ou de débit de dose suffisamment élevés pour délivrer une dose de rayonnement ionisant avec précision en des instants très brefs.

Particulièrement, un autre problème technique majeur de ces dispositifs est que le détecteur utilisé n'est pas rapide, ce qui rend impossible la détection de la dose à des échelles de temps très court, par exemple de l'ordre de la nanoseconde.

Particulièrement, un autre problème technique majeur de ces dispositifs est que le détecteur utilisé sature à partir d'un certain débit de dose, généralement dès 10 Gy/s au mieux, ce qui rend impossible la détection de débits de dose très élevés, par exemple de l'ordre de 250 Gy/s, voire 500 Gy/s ou même 1000 Gy/s.

Un autre problème technique qui se pose est que les moyens de contrôle de dose et les moyens de contrôle et de commande ne sont pas efficaces pour exécuter de façon souhaitée et de matière très précise, la délivrance d'une dose prescrite en un temps très bref.

Pour obtenir une bonne précision de la dose délivrée au cours d'une irradiation d'une durée de quelques millisecondes ou de l'ordre de la seconde, il suffit que les durées qui séparent le début de la détection par l'électronique de contrôle et de commande du début de l'émission par l'accélérateur d'une part et le moment de la détection par l'électronique de contrôle et de commande du dépassement de la dose prescrite de l'arrêt de l'émission par l'accélérateur d'autre part, soient inférieures à une milliseconde, de préférence inférieure à quelques microsecondes.

Les problèmes techniques évoqués précédemment conduisent à une incapacité à délivrer et/ou à mesurer et/ou à contrôler précisément de fortes doses de rayonnement ionisant en des durées très brèves. Les impacts d'un mauvais contrôle de dose et/ou du débit de dose absorbée par la cible peuvent conduire à la destruction pure et simple des cellules, tissus ou organes sains et les effets secondaires qui en découlent peuvent avoir dans certains cas, des conséquences néfastes sur les organes à risques.

De l'état de la technique est connu des dispositifs d'irradiation à rayonnement ionisant capables de délivrer des débits de dose aussi élevés que 10 kGy/s mais en électrons seulement et à des énergies inférieures ou égales à 10 MeV.

Il est également connu de l'état de la technique des accélérateurs comprenant des systèmes de contrôle pour inhiber une irradiation en moins de 100 $\mu$s, mais leur système de détection à chambre d'ionisation n'est pas suffisamment rapide pour détecter le débit de dose, l'intégrer, le comparer et arrêter l'irradiation en moins de 100 ms.

Aujourd'hui, il semble impératif de concevoir une architecture d'un dispositif d'irradiation à rayonnement ionisant notamment pour la radiothérapie et/ou la radiobiologie permettant de délivrer de manière contrôlée et précise de fortes doses de rayonnement ionisant d'au moins 0,25 Gy avec une précision d'au moins 1 $\mu$Gy, de préférence 1 nGy, en des instants très brefs, c'est-à-dire d'au moins 0,1 $\mu$s, notamment 100 $\mu$s, voire 1 ms, voire 100 ms, dans des gammes d'énergie comprises entre 1 MeV et 50 MeV.

De la publication WO 2007 017177 est connu un dispositif de radiothérapie comprenant une source pulsée d'électrons, capable de fournir une exposition totale pendant au moins une minute à la fréquence de répétition de 1 Hz et de fournir des doses uniques de 10 Gy en environ 30 ns.

De la publication US 2010 329413 est connu un dispositif de radiothérapie comprenant un système et un procédé capable de délivrer des rayons X avec un débit de dose pouvant atteindre environ 10 Gy/s (gray par seconde) voire sensiblement plus.

De la publication US 6445766 est également connu un dispositif de radiothérapie comprenant des moyens pour produire un rayonnement ionisant.

De la publication US 7 567647 et US 2008 144772 est connu un dispositif de radiothérapie comprenant un moyen d'émission de rayonnement ionisant, une cible, un détecteur de dose, un moyen de contrôle de la dose.

Cependant, aucun des documents cités ne divulgue une boucle de régulation et de contrôle spécifique reliant intelligemment les différents organes composant le dispositif d'émission de rayonnement ionisant pour délivrer de façon efficace une dose d'au moins 1 Gy, notamment d'au moins 10 Gy dans des gammes d'énergie comprises entre 1 MeV et 25 MeV et de préférence jusqu'à 50 MeV, en des instants très brefs, c'est-à-dire d'au moins 0,1 $\mu$s, de préférence 100 $\mu$s, ou 1 ms, voire 100 ms.

Egalement, aucun des documents cités ne divulgue un détecteur capable de détecter une dose en des temps très courts, de préférence d'au moins 0,01 ns, c'est-à-dire un détecteur ultra-rapide.

De plus, aucun des documents cités ne satisfait toutes les spécifications pour délivrer un débit de dose de rayonnement ionisant jusqu'à 250 Gy/s, voire jusqu'à 500 Gy/s ou même encore jusqu'à 1000 Gy/s, dans une gamme d'énergie comprise entre 1 MeV et 25 MeV, voire 50 MeV, de manière contrôlée pendant des durées de temps inférieures à 1 s, notamment 1 ms.

Le but de la présente invention est de fournir un dispositif d'irradiation à rayonnement ionisant notamment pour la radiothérapie et/ou radiobiologie remédiant aux problèmes précédemment évoqués et améliorant les dispositifs d'irradiation à rayonnement ionisant connus de l'état de la technique.

Dans la description qui suit, les termes listés ci-après auront la définition suivante :

- Dose absorbée : la dose absorbée, ou, plus concisément dose de rayonnement ionisant ou la dose, est l'énergie déposée par unité de masse de matière soumise à du rayonnement ionisant. La dose absorbée mesure la densité massique d'énergie déposée par irradiation. L'unité de dose dans le système international est le gray (Gy) ; c'est une unité dérivée valant un joule par kilogramme : 1 Gy = 1 J/kg. Considérant un faisceau de rayonnement ionisant irradiant un élément de volume dV, de masse volumique p et de masse dm = pdV. Soit dE l'énergie déposée dans cet élément par le faisceau, la dose absorbée D est définie par :

$$D = \frac{\mathrm{d}E}{\mathrm{d}m} = \frac{1}{\rho}\frac{\mathrm{d}E}{\mathrm{d}V}$$

- Débit de dose absorbée : dose absorbée par la matière soumise à un rayonnement ionisant, par unité de temps. Il se mesure ein Gy/s (grays par seconde) dans le système d'unités international.
- Electronvolt : de symbole eV, est une unité de mesure d'énergie. Sa valeur est définie comme étant l'énergie acquise par un électron accéléré par une différence de potentiel d'un volt : 1 eV = e·(1 V), où e est la valeur absolue de la charge de l'électron.
- Méga-électron-volt: de symbole MeV, 1 MeV = 106 eV = 1,6022.10-13 J.
- Nanoseconde : Unité de mesure de temps du système international, valant 10-9 seconde, et dont le symbole est ns.
- Microseconde : Unité de mesure de temps du système international, valant 10-6 seconde, et dont le symbole est $\mu$s.
- Milliseconde : Unité de mesure de temps du système international, valant 10-3 seconde, et dont le symbole est ms.
- Seconde : Unité de mesure de temps du système international dont le symbole est s.
- Détection très précise : mesure d'une grandeur dont l'incertitude relative du résultat est très petite, d'au plus 0,01%.
- Rayonnement ionisant : rayonnement capable de déposer assez d'énergie dans la matière qu'il traverse pour créer une ionisation, c'est-à-dire ioniser des atomes et/ou des molécules qui constituent ladite matière.
- Forte dose : dose de rayonnement ionisant qui, selon le type de rayonnement ionisant, produit des effets déterministes sur un organisme vivant ou bien qui est supérieure à ce qu'une cible peut recevoir au cours de sa vie par les rayonnements ionisants naturels et ceux dus à l'activité humaine. Quel que soit le type de rayonnement ionisant, une forte dose est caractérisée par une dose absorbée supérieure à 0,01 Gy.
- Interconnexion intelligente : c'est une interconnexion qui utilise des technologies informatiques de manière à optimiser le transfert et la distribution d'informations, et qui a pour objectif d'optimiser l'ensemble des mailles d'un réseau d'électricité afin d'améliorer l'efficacité de transfert et la réponse à une sollicitation donnée.
- Une valeur souhaitée : d'une manière générale, c'est une valeur choisie, définie et programmée par un opérateur via l'interface homme machine.
- Contrôle intelligent : c'est un contrôle qui utilise des technologies informatiques de manière à optimiser la valeur d'une grandeur à contrôler, le transfert et la distribution d'informations, et qui a pour objectif d'optimiser le contrôle de l'ensemble des mailles d'un réseau d'électricité afin d'améliorer l'efficacité de transfert et la réponse à une sollicitation donnée.

L'invention est définie dans les revendications attenantes.

L'invention a pour objet un dispositif d'irradiation à rayonnement ionisant, notamment pour la radiothérapie et/ou la radiobiologie, comprenant simultanément au moins :

- un moyen d'émission de rayonnement ionisant (MER) comprenant :

    ○ au moins une source de particules qui comprend au moins :

        ▪ une cathode et une anode, ou un plasma,
        ▪ un moyen de déclenchement d'émission de particules, notamment une grille ou une électrode ou un laser et
        ▪ un moyen d'accélération d'un faisceau de particules, ○ ledit moyen d'émission de rayonnement ionisant (MER) étant aussi doté d'un système de contrôle d'impulsion de puissance qui est configuré pour produire un faisceau de particules, c'est-à-dire un rayonnement ionisant, d'énergie, ajustable et souhaitée, dans une gamme comprise entre 1 MeV et 50 MeV, pulsé à une fréquence (f) souhaitée, typiquement comprise entre 5 Hz et 1000 Hz, voire entre 5 Hz et 500 Hz, voire entre 5 Hz et 200 Hz, et de préférence d'environ 100 Hz, avec une durée d'impulsion (d), ajustable, d'au moins 1 ns, et de préférence comprise entre 0,05 $\mu$s et 12 $\mu$s, et de préférence de 0,1 $\mu$s, et pour délivrer un débit de dose absorbé d'au moins environ 0,01 Gy/s, par exemple compris entre environ 0,01 Gy/s et environ 250 Gy/s, voire 500 Gy/s ou même encore 1000 Gy/s,

- un moyen de détection de dose de rayonnement ionisant (MDD) comprenant un détecteur ; le détecteur étant un détecteur ultra-rapide, couplé à un moyen de contrôle de dose (MCD), configuré pour détecter, de façon très précise, une dose de rayonnement ionisant en des temps très courts, c'est-à-dire en au moins 0,01 ns, et à des débits de dose très élevés c'est-à-dire permettant de délivrer au moins 0,01 Gy/s, par exemple 25 Gy/s voire 50 Gy/s, voire de préférence 250 Gy/s, voire encore 500 Gy/s ou même encore 1000 Gy/s,
- le moyen de contrôle de dose (MCD), configuré pour contrôler un déclenchement et un arrêt du moyen d'émission de rayonnement ionisant (MER), comprenant :

o une électronique de contrôle (EC), configurée pour contrôler une dose délivrée pendant une fraction de seconde, c'est-à-dire pendant au moins 0,1 μs, voire 100 μs, voire 1 ms, voire encore 100 ms,

∘ un amplificateur et/ou un atténuateur amplifiant et/ou atténuant un signal émis par le détecteur,

∘ un intégrateur intégrant pendant la durée de l'émission ledit signal amplifié et/ou atténué et

∘ un comparateur comparant continument ledit signal intégré aux consignes de la dose prescrite (CDA) prédéterminée par un opérateur,

- un système de contrôle et de commande (SCC), comportant :

∘ un système de contrôle fonctionnel (SCF) des différents organes dudit dispositif, comprenant :

▪ une interface homme machine (IHM),
▪ des outils de visualisation et
▪ des moyens de mise en oeuvre configurés pour qu'un opérateur programme, de façon contrôlée et souhaitée et de façon très précise, en une seule fois, la nature du rayonnement, le débit de dose absorbé de rayonnement ionisant, une durée et un régime impulsionnel de l'émission du rayonnement ionisant.

Lesdits moyens (MER, MDD, MCD) et ledit système (SCC), constituant au moins en partie ledit dispositif et étant en outre interconnectés entre eux de façon intelligente pour coopérer et former une boucle de contrôle et de régulation intelligente, notamment de la puissance, sont configurés délivrer et contrôler des doses de rayonnement ionisant d'au moins 0,01 Gy voire 0,25 Gy avec une précision d'au moins 1 μGy, de préférence 1 nGy, à des énergies comprises entre 1 MeV et 50 MeV, pendant des instants très brefs, c'est-à-dire d'au moins 0,1 μs, voire 100 μs, de préférence 1 ms, voire 100 ms, ou par exemple compris entre 0,1 μs et 100 ms, de préférence 100 μs ou 1 ms.

Selon d'autres caractéristiques de l'invention, le moyen d'émission de rayonnement ionisant (MER) est un accélérateur de particules qui comprend une source de puissance et une électronique de contrôle, c'est-à-dire un système de contrôle d'impulsion, ladite électronique de contrôle (ou système de contrôle d'impulsion) est directement reliée au moyen de contrôle de dose (MCD) de façon à arrêter automatiquement la source de puissance lorsque la dose absorbée, détectée et mesurée a atteint une valeur prescrite et prédéterminée par l'opérateur via l'interface homme machine (IHM), ladite interface homme machine (IHM) comprend en outre un poste de commande (PC) et un programme de gestion d'interface de visualisation.

Selon d'autres caractéristiques de l'invention, le moyen d'accélération est soit à onde hyperfréquence, soit à induction, soit électrostatique.

Avantageusement, le moyen d'émission de rayonnement (MER) comprend en outre plusieurs moyens d'accélérations montés en série et/ou intercalés de moyens de déviation ou de recirculation du faisceau de particules à travers le ou lesdits moyens d'accélération, chacun étant alimenté par au moins une source de puissance.

Avantageusement, le détecteur est un semi-conducteur, notamment du diamant, par exemple sous forme monocristalline ou poly-cristalline, pur ou dopé, ou du carbure de silicium, compris entre deux électrodes de polarisation aux bornes desquelles est appliquée une tension de quelques volts réglable selon l'épaisseur du détecteur via l'interface homme-machine, et configuré pour obtenir un temps de réponse très court, par exemple d'au moins 0,01 ns, à de forts débits de dose, c'est-à-dire d'au moins 0,01 Gy/s.

Traditionnellement, les détecteur à semi-conducteur étaient utilisés pour des gammes d'énergie bien plus faible que dans le cadre de la présente invention, typiquement sous 1 MeV et sont connus pour avoir comme inconvénients de présenter une sensibilité variable, les rendant particulièrement mal adaptés au domaine médical.

Pourtant, dans le cadre de la présente invention, le détecteur est traversé par le faisceau et est configuré pour permettre une surveillance de la dose délivré au patient pendant le traitement. Il détecte donc l'intégralité du flux de rayonnement ionisant en temps réel. On pouvait donc s'attendre à ce que les détecteurs connus fournissent des informations difficiles à interpréter, d'une part, et surtout saturent dans la gamme de doses considérées, mais il est apparu de manière surprenante que, contrairement au préjugé de l'homme de métier, des détecteurs de ce type permettaient, tout en étant traversés par l'intégralité du flux de rayonnement ionisant destiné à être appliqué à un patient, de donner une valeur significative de ce flux, de manière à la fois précise et rapide.

Un détecteur en diamant prometteur dans le cadre de la présente invention est par exemple développé par le laboratoire LCD (Laboratoire Capteur Diamant) du CEA de Saclay.

Pour un détecteur en carbure de silicium, un de ceux étudiés et développés par le laboratoire IM2NP (Institut Matériaux Microélectronique Nanosciences De Provence, UMR CNRS 7334), qui est spécialisé dans le carbure de silicium, procure de premiers résultats intéressants.

Avantageusement, l'électronique de contrôle (EC) du moyen de contrôle de dose (MCD) comprend des moyens de mesure de courant électrique, notamment d'un signal électrique, produit par l'interaction du rayonnement avec le détecteur, des moyens de conversion dudit courant électrique en unité de débit de dose absorbée et des moyens d'intégration dudit courant électrique configurés pour mesurer avec précision la dose absorbée cumulée au cours de l'émission du rayonnement ionisant.

Avantageusement, un signal électrique est produit par le détecteur, ledit signal électrique est mesuré et intégré par un électromètre au cours de l'émission du rayonnement, la valeur intégrée dudit signal électrique est directement comparée à la valeur de la dose prescrite et prédéterminée par l'opérateur via l'interface homme machine de façon à ce que, dès que la valeur intégrée est supérieure ou égale à la valeur prescrite et prédéterminée, le signal du système de déclenchement d'émission de particules est interrompu pour arrêter, interdire, instantanément l'émission du rayonnement et, éventuellement, de préférence la source haute tension en un temps suffisamment court, par exemple d'au moins 1 ns.

Selon d'autres caractéristiques de l'invention, le détecteur comprend un ou plusieurs quadrants ou secteurs ou voxels produisant chacun un signal de détection ou signal de dose absorbée (SDA) et disposés intelligemment, c'est-à-dire configurés pour permettre de déduire de leurs signaux de détection (SDA) des informations caractérisant la position, la forme et/ou l'énergie du faisceau de rayonnement ionisant qui les traverse d'une part, et de façon à contrôler et réguler le faisceau de rayonnement ionisant, notamment sa position, sa forme et/ou son énergie d'autre part.

D'autres caractéristiques et avantages de l'invention pris seuls ou en combinaison, apparaîtront à la lecture de la description détaillée qui suit pour la compréhension de laquelle on se reportera aux dessins annexés dans lesquels :

- la figure 1 est une représentation schématique d'un principe général de fonctionnement des différents moyens et organes du dispositif selon l'invention définissant une boucle de régulation et de contrôle,
- la figure 2 présente schématiquement un exemple de réalisation d'un dispositif selon l'invention,
- la figure 3 illustre certains des principaux paramètres d'une dose, à savoir la durée du tir (D), la durée d'une impulsion (d) et la fréquence d'impulsion (f), et
- la figure 4 est une représentation schématique d'un exemple de moyen d'émission de rayonnement ionisant (MER).

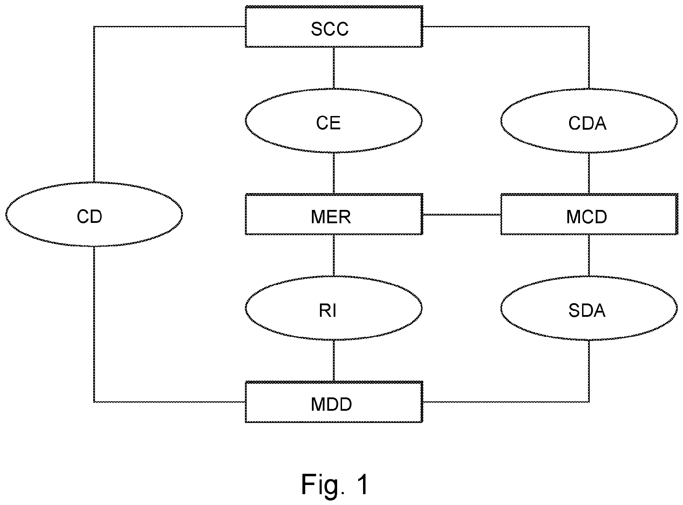

La figure 1 montre un descriptif général du principe d'interconnexion intelligente des différents organes composant le dispositif d'irradiation à rayonnement ionisant pour la radiothérapie et/ou la radiobiologie objet de la présente invention. Ce principe montre que les différents organes et/ou sous-organes constituant le dispositif selon l'invention coopèrent ensemble et forment une boucle de contrôle et de régulation, notamment de contrôle et de régulation de puissance de façon à délivrer de manière contrôlée et précise de fortes doses de rayonnement ionisant d'au moins 0,25 Gy avec une précision d'au moins 1 $\mu$Gy, de préférence 1 nanogray (nGy), dans une gamme d'énergie comprise entre 1 MeV et 50 MeV, en des durées très brèves, c'est-à-dire par exemple comprises entre 0,1 $\mu$s et 100 ms, de préférence 100 $\mu$s ou 1 ms. Cette coopération et interconnexion et la boucle formée sont essentielles pour obtenir les fonctions techniques objet de la présente invention. L'association des consignes techniques, notamment électroniques à chaque niveau de la boucle de contrôle et de régulation montre combien de fois cette interconnexion est nécessaire pour la réalisation de l'invention et permet de contrôler de façon précise et sans ambigüité, le fonctionnement du dispositif et de la dose délivrée.

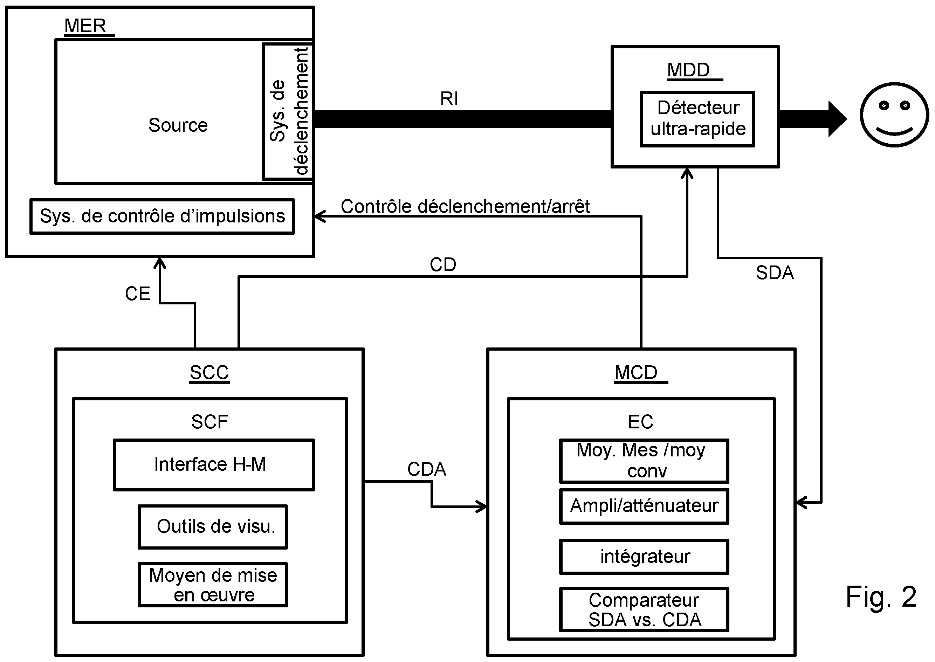

En référence aux figures 1 et 2, le dispositif comprend quatre éléments interconnectés entre eux : au moins un moyen d'émission de rayonnement ionisant (MER), un moyen de détection de la dose (MDD), un système de contrôle et de commande (SCC) et un moyen de contrôle de la dose (MCD).

Le système de contrôle et de commande (SCC) est programmé par un opérateur via un logiciel d'interface de façon à envoyer, en temps réel, des signaux de consignes d'émission (CE) au moyen d'émission de rayonnement ionisant (MER), des signaux de consignes de détection (CD) au moyen de détection de dose de rayonnement ionisant (MDD) et des signaux de consignes de dose absorbée (CDA) au moyen de contrôle de dose de rayonnement ionisant (MCD).

Egalement, le moyen d'émission de rayonnement ionisant (MER) émet un rayonnement ionisant (RI) correspondant au signal de rayonnement ionisant qui interagit avec le moyen de détection de dose (MDD).

L'interaction du rayonnement ionisant (RI) avec le moyen de détection de dose (MDD) génère l'envoi d'un signal de dose absorbée (SDA), par le moyen de détection de dose (MDD) vers le moyen de contrôle de dose (MCD). Ledit moyen de contrôle de dose (MCD) amplifie ou attenue, intègre et compare le signal de dose absorbée (SDA) au signal de consigne de dose absorbée (CDA). Si le résultat de l'amplification ou de l'atténuation et de l'intégration du signal de dose absorbée (SDA) est supérieur ou égal au signal de consigne de dose absorbée (CDA), le moyen de contrôle de dose absorbée (MCD) envoie un signal qui commande l'interruption du moyen d'émission de rayonnement ionisant (MER), sinon l'émission de rayonnement ionisant (RI) se poursuit selon les prescriptions prédéfinies par l'opérateur.

La disposition des différents organes de consigne, de contrôle et leurs interconnexions constituant la boucle de contrôle et régulation fait de sorte qu'il est possible de délivrer de manière contrôlée et précise, de façon intelligente, de fortes doses de rayonnement ionisant d'au moins 0,25 Gy avec une précision d'au moins 1 $\mu$Gy, de préférence 1 nGy, dans une gamme d'énergie comprise entre de 1 MeV et 50 MeV, pendant des instants très brefs, c'est-à-dire par exemple compris entre environ 0,1 $\mu$s et 100 ms, de préférence 100 $\mu$s ou 1 ms.

Le moyen de détection de la dose (MDD) comprend particulièrement le détecteur ultra-rapide capable de détecter, de façon très précise, une dose en des temps très courts, par example d'au moins 0,01 ns, couplé à un moyen de contrôle de dose (MCD), lequel comprend une électronique de contrôle (EC). Ladite électronique de contrôle (EC) est capable de contrôler une dose délivrée d'au moins 0,01 Gy, voire au moins 0,25 Gy pendant au moins 0,1 μs, voire 100 μs, voire 1 ms, voire 100 ms.

Le détecteur ultra-rapide du moyen de détection de la dose (MDD) est notamment en diamant et est compris entre deux électrodes de polarisation aux bornes desquelles est connecté l'électronique de contrôle (EC) du moyen de contrôle de la dose (MCD), qui mesure le signal électrique de détection (SDA), notamment le courant, produit par le rayonnement ionisant à travers le diamant, le converti en unité de monitorage de débit de dose absorbée et l'intègre pour donner une mesure de dose absorbée cumulée au cours de l'émission de rayonnement. L'électronique de conversion du signal électrique produit par l'interaction du diamant et du rayonnement ionisant est par exemple calibrée à l'aide d'un instrument de mesure absolue de dose absorbée cumulée pendant une émission de rayonnement ionisant.

Selon d'autres caractéristiques particulièrement intéressantes de l'invention, le détecteur ultra-rapide comprend un ou plusieurs détecteurs dits « primaires » qui comporteraient chacun plusieurs petits détecteurs qualifiés de secondaires, c'est-à-dire un ou plusieurs quadrants ou secteurs ou voxels produisant chacun un signal de détection (SDA) et disposés de façon à déduire de leurs signaux de détection (SDA) des informations caractérisant la position, la forme et/ou l'énergie du faisceau de rayonnement ionisant qui les traverse d'une part, et de façon à contrôler et réguler le faisceau de rayonnement ionisant, notamment sa position, sa forme et/ou son énergie d'autre part. Par exemple, il comporte quatre quadrants ou secteurs ou voxels disposés en carré.

Par exemple, chaque détecteur qualifié de détecteur primaire comprend plusieurs zones de détections ou plusieurs petits détecteurs qualifiés de secondaires. Le détecteur ultra-rapide peut être un empilement de détecteurs primaires sous forme de disques de matériau semi-conducteur, séparés en plusieurs détecteurs secondaires.

Avantageusement, le détecteur est un semi-conducteur, notamment le diamant, compris entre deux électrodes de polarisation aux bornes desquelles est appliquée une tension de quelques volts réglable via l'interface homme-machine, ce qui permet d'obtenir un temps de réponse très court, par exemple d'au moins 0,01 ns, à des forts débits de dose, c'est-à-dire d'au moins 0,01 Gy/s. Selon un autre exemple de réalisation intéressant, ledit détecteur peut être réalisé en carbure de silicium. Le semi-conducteur, notamment le diamant, est compris entre deux électrodes de polarisation aux bornes desquelles est appliquée une tension de polarisation de quelques volts (TP) par exemple un volt par micromètre d'épaisseur de diamant entre les électrodes.

Comme décrit précédemment, la tension de polarisation (TP) est réglable par le système de contrôle et de commande (SCC) via l'interface homme machine. Aux bornes des électrodes de polarisation est connecté un moyen d'interconnexion de polarisation qui permet à la fois d'apporter la tension de polarisation (TP) commandée par le système de contrôle et de commande (SCC) et de transmettre le signal de dose absorbée (SDA) produit par le diamant par interaction avec le rayonnement ionisant au moyen de contrôle de dose (MCD).

Avantageusement, le moyen de contrôle de dose (MCD) comprend des moyens de mesure du signal électrique (SDA), notamment de mesure de courant, produit par le rayonnement émis à travers le détecteur, notamment par interaction entre le détecteur et le rayonnement ionisant qui le traverse, des moyens de conversion dudit signal électrique de détection (SDA), notamment de courant, en unité de monitorage de débit de dose absorbée et des moyens d'intégration dudit signal électrique de détection (SDA), notamment de courant, de façon à mesurer avec précision la dose absorbée cumulée au cours de l'émission du rayonnement ionisant.

Le détecteur choisi offre l'intérêt d'avoir un temps de réponse très court de l'ordre de la nanoseconde ou encore même d'un ordre inférieur à la nanoseconde. Il offre également une résistance élevée au rayonnement ionisant, des propriétés d'interactions avec le rayonnement ionisant similaires à celle de l'eau, c'est-à-dire que l'énergie déposée par le rayonnement ionisant en le traversant est comparable à celle que le même rayonnement déposerait dans de l'eau, la conversion d'une telle énergie en signal électrique de détection (SDA) rendant ce dernier représentatif de la dose déposée par le rayonnement ionisant dans la matière vivante, typiquement un patient. Il offre également une réponse linéaire en courant du signal électrique par rapport au débit de dose sur de larges intervalles de forts débits de dose, et/ou à la durée d'impulsion (d) du rayonnement ionisant. Il offre aussi une réponse en courant constant par rapport à l'énergie du rayonnement ionisant.

Ces diverses propriétés physiques et éventuellement chimiques font notamment du diamant un matériau approprié à la mesure de fort débit de dose de rayonnement ionisant, notamment de débit de dose d'au moins 25 Gy/s voire d'au moins 50 Gy/s, en impulsions de rayonnement ionisant très brèves de l'ordre de 100 ns, pour une longue durée de vie, c'est-à-dire d'au moins 500 heures.

Le système de contrôle et de commande (SCC) est doté d'un système de contrôle fonctionnel des différents organes dudit dispositif selon l'invention. Ce système de contrôle et de commande (SCC) comprend principalement l'interface homme machine (IHM) comprenant plusieurs boutons et organes de gestion et de programmation du dispositif, notamment en matière de dose ou de débit de dose absorbée ou de débit de dose délivrée de rayonnement ionisant. Le système de contrôle et de commande (SCC) comprend en outre des outils de visualisations et plusieurs moyens secondaires de commande, notamment des moyens tactiles et non tactiles, des interrupteurs, etc, nécessaires pour la mise en oeuvre du dispositif. Ces moyens permettent à un opérateur de programmer de façon contrôlée et souhaitée et de façon très précise, de manière intelligente, en une seule fois, la nature du rayonnement, la dose absorbée et/ou le débit de dose absorbée et éventuellement le débit de dose délivrée, de rayonnement ionisant, en une durée souhaitée, et le régime impulsionnel.

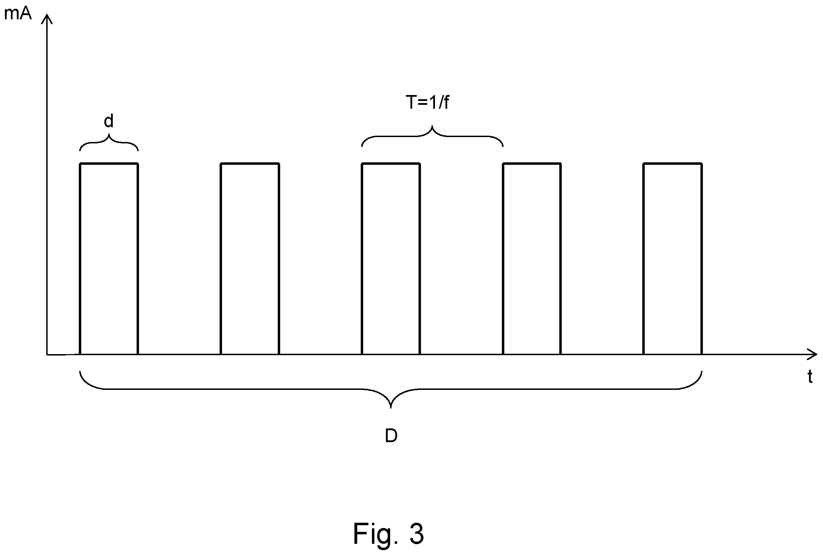

Les paramètres d'impulsion de grille, amplitude, fréquence (f) et durée d'impulsion (d), nombre d'impulsions par train d'impulsions ou durée de train d'impulsions, durée entre trains d'impulsions et le nombre de train d'impulsions ou durée totale d'émission (D) (comme schématisés sur la figure 3) sont définis par l'opérateur via l'interface homme-machine et un logiciel adapté. Ces paramètres sont transmis à une électronique programmée pour générer des signaux de synchronisation des impulsions hautes fréquences (HF) de l'accélérateur et des impulsions de grille de canon à électrons ou de déclenchement de source de faisceau de particules. Les signaux sont amplifiés par une électronique basse tension pour produire des impulsions de grille et par une électronique de puissance pour la production d'impulsion haute fréquence (HF).

Par ailleurs, le détecteur, comme décrit précédemment, est placé dans le faisceau de rayonnement ionisant pour mesurer le débit de dose absorbée que ce rayonnement ionisant peut produire dans la matière avec laquelle il interagit, typiquement un patient. Le signal (SDA) produit par le détecteur sous forme d'un courant électrique est mesuré et intégré par l'électronique de contrôle (EC) du moyen de contrôle de dose (MCD), notamment un électromètre, au cours de l'émission du rayonnement ionisant et la valeur intégrée est comparée à la valeur de la dose absorbée prescrite (CDA) par l'opérateur via l'interface homme-machine (IHM). Dès que la valeur intégrée est supérieure ou égale à la valeur prescrite, le signal d'impulsion de grille retombe à sa tension de polarisation négative par rapport à la cathode de façon à interdire l'émission. La durée d'émission de rayonnement ionisant peut donc être contrôlée et stoppée en temps réel et la dose délivrée est précise à la valeur près de la dose produite par la dernière impulsion de rayonnement ionisant.

La fonction de mesure du signal de détection peut par exemple être assurée par un amplificateur ou un atténuateur selon l'amplitude du signal, suivi d'un échantillonneur-bloqueur. Le moyen de contrôle de dose (MCD) est un moyen configuré pour contrôler le déclenchement et l'arrêt du moyen d'émission de rayonnement ionisant (MER), il comprend l'électronique de contrôle et de commande (EC). Dans un exemple de réalisation particulièrement commode, l'électronique de contrôle et de commande (EC) comprend l'amplificateur ou atténuateur amplifiant ou atténuant, selon l'amplitude, un signal émis par le détecteur, l'intégrateur intégrant pendant la durée de l'émission ledit signal amplifié ou atténué et le comparateur comparant continument ledit signal intégré aux consignes de dose prescrite (CDA) prédéterminée par l'opérateur.

Pour commander l'émission de rayonnement ionisant pendant des instants très brefs, l'émetteur (MER) est constitué d'une source de puissance qui est déclenchée et arrêtée par l'électronique du système de contrôle et de commande (SCC). L'opérateur commande le déclenchement d'une émission programmée via l'interface homme-machine (IHM). L'électronique du système de contrôle et de commande (SCC) couplé au moyen de contrôle de dose (MCD) arrête les sources de puissance lorsque la dose absorbée, détectée et mesurée à atteint la valeur prescrite et prédéterminée par l'opérateur.

Grace à une électronique rapide du moyen de contrôle de dose (MCD), lorsque le signal amplifié ou atténué et intégré issu du détecteur (SDA) égalise le signal de consigne de la dose (CDA), automatiquement le signal de sortie du comparateur tombe à une valeur prédéterminée et prédéfinie et déclenche l'arrêt de la source du faisceau d'électrons, notamment du moyen d'émission de rayonnement ionisant (MER), et l'arrêt de la source de haute tension (HT) de l'accélérateur en un temps suffisamment court, c'est-à-dire de préférence inférieur à 100 $\mu$s de façon à ce que la dose détectée ne dépasse pas la dose prescrite.

Tous les moyens précédemment cités constituant la boucle de contrôle et de régulation, notamment en puissance, constituant le dispositif objet de la présente invention, sont interconnectés entre eux de façon intelligente comme décrit précédemment, pour coopérer et former une boucle de contrôle et de régulation intelligente, notamment de la puissance, de façon à délivrer de manière contrôlée et précise de fortes doses de rayonnement ionisant, c'est-à-dire de préférence d'au moins 0,01 Gy et de préférence d'au moins 0,25 Gy avec une précision d'au moins 1 $\mu$Gy, de préférence 1 nGy, dans une gamme d'énergie comprise entre 1 MeV et 50 MeV, en des instants très brefs, c'est-à-dire par exemple compris entre environ 0,1 $\mu$s et 100 ms, de préférence 100 $\mu$s ou 1 ms.

Avantageusement le moyen d'émission de rayonnement ionisant (MER) est constitué principalement de l'accélérateur de particules comprenant une source de puissance et du système de contrôle d'impulsion qui est par exemple directement reliée au moyen de contrôle de la dose (MCD) de façon à arrêter automatiquement la source de puissance lorsque la dose absorbée, détectée et mesurée a atteint la valeur prescrite et prédéterminée par l'opérateur via l'interface homme-machine (IHM).

Ladite interface homme machine (IHM) comprend en outre un poste de commande (PC) et un programme de gestion d'interface de visualisation. L'interface homme machine comprend également des boutons poussoirs et des voyants lumineux équipant les baies, les boitiers et/ou des cartes électroniques qui contiennent les électroniques du système de contrôle et de commande (SCC) et du moyen de contrôle de dose (MCD).

Avantageusement, lors du fonctionnement du dispositif, un signal est produit par le détecteur sous forme de courant électrique, ledit signal est mesuré et intégré par un électromètre au cours de l'émission du rayonnement, la valeur intégrée dudit signal est directement comparée à la valeur de la dose prescrite et prédéterminée par l'opérateur via l'interface homme-machine (IHM) de façon à ce que, dès que la valeur intégrée est supérieure ou égale à la valeur prescrite et prédéterminée, le signal d'impulsion de la grille retombe automatiquement à une valeur prédéterminée correspondant à la tension de polarisation par rapport à la cathode pour interdire et arrêter instantanément l'émission du rayonnement ionisant et éventuellement la source haute tension en un temps suffisamment court, par exemple d'au moins 1 ns.

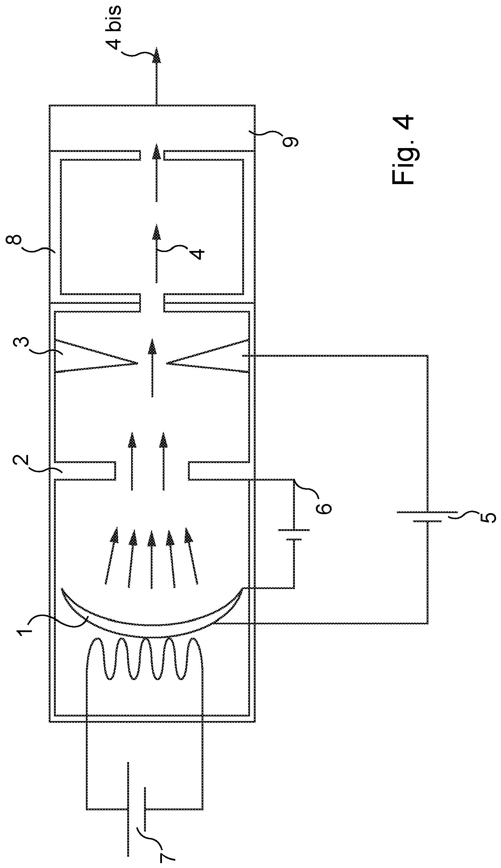

La figure 4 est un mode de réalisation représentant un moyen d'émission de rayonnement ionisant (MER). Ce moyen d'émission de rayonnement ionisant (MER) comprend au moins une source de particules, notamment d'électrons. Ladite source comprend par exemple au moins une cathode (1), une anode (3) et une grille (2). La grille comprend en outre une polarisation à basse tension (6) qui contrôle l'extraction et le débit de particules extraites de la cathode.

Le moyen d'émission de rayonnement ionisant (MER) comprend en outre un filament (7), alimenté en tension, notamment en basse tension, pour chauffer la cathode et la rendre ainsi émissive, c'est-à-dire, pour pouvoir en extraire des particules.

La grille est configurée pour que les particules extraites de la cathode traversent ladite grille. La cathode et l'anode sont connectées à une alimentation haute tension HT (5). Ainsi, les particules ayant traversé la grille sont accélérées vers l'anode en formant un faisceau de particules.

La source de faisceau de particules peut être par exemple une source déclenchable par un faisceau laser, ou une source déclenchable par une grille polarisée ou par une électrode polarisée. Dans ce premier cas, la source est composée d'une photocathode ou d'un plasma, d'une anode et d'un faisceau laser qui illumine la photocathode ou le plasma pour déclencher l'émission d'électrons par la photocathode ou par le plasma. L'anode est pourvue d'un orifice permettant l'extraction du faisceau de particules et son injection dans un moyen d'accélération (8) du faisceau de particules (4).

Le moyen d'émission de rayonnement ionisant (MER) comporte en outre d'une enceinte à vide, c'est-à-dire à une très faible pression, par exemple d'au plus $10^{-6}$ mbar.

Le moyen d'émission de rayonnement ionisant (MER) comprend en outre une fenêtre de transmission ou une cible de conversion (9) qui converti le faisceau de particules (4) en rayonnement ionisant (4 bis) par transmission depuis l'enceinte à vide vers l'atmosphère extérieure. C'est de cette manière qu'est émis le rayonnement ionisant.

De façon générale, le principe général d'émission de particules ou de rayonnement ionisant est largement connu de l'état de la technique.

Le moyen d'émission de rayonnement ionisant (MER) est doté d'un système de contrôle d'impulsion de puissance qui comprend un générateur de puissance alimentant le moyen d'accélération de faisceau de particules et une électronique de commutation. Ledit système de contrôle d'impulsion de puissance est capable de produire un faisceau de particules d'énergie ajustable et souhaitée dans une gamme comprise entre 1 MeV et 50 MeV, pulsé à une fréquence (f) souhaitée, typiquement entre 5 Hz et 1 kHz, avec une durée d'impulsion (d) ajustable d'au moins 1 ns, de préférence 0,1 μs et capable de délivrer un débit de dose absorbée jusqu'à 250 Gy/s, voire jusqu'à 500 Gy/s ou même encore jusqu'à 1000 Gy/s.

A énergie ajustée et souhaitée, le débit de particules, autrement dit le courant du faisceau de particules, est commandé par la tension de polarisation (6) appliquée à la grille. C'est la combinaison de l'énergie et du courant du faisceau de particules qui déterminent le débit de dose absorbée que peut délivrer le moyen d'émission de rayonnement ionisant (MER).

Le moyen d'émission de rayonnement ionisant est doté en outre d'une source de faisceau de particules déclenchée par la grille (2). Le courant de faisceau de particules généré par la source à grille est directement fonction de la tension de polarisation (6) de ladite grille (2), notamment sous forme d'un signal ayant une durée d'impulsion (d) d'au moins 1 ns, de préférence 0,1 μs, une fréquence d'impulsion (f) et une amplitude de tension de polarisation.

L'électronique de commutation alimentant ladite grille peut fonctionner en plusieurs modes, notamment en mode récurrent pour l'irradiation de longue durée, en mode mono-impulsion pour les irradiations de très courtes durées inférieures à de préférence 1 ms, voire 100 μs, et en mode semi-récurrent pour les irradiations composée de plusieurs trains d'impulsions, de fréquences choisies et séparés en fonction de délais choisis.

Idéalement, la forme du signal de polarisation de la grille peut être programmée à volonté avec des impulsions de durée variable, à fréquence variable, d'amplitude variable et avec au moins un délai prédéfini entre impulsions et/ou entre trains d'impulsions.

L'électronique de commutation du système de contrôle d'impulsion de puissance du moyen d'émission de rayonnement (MER) comprend des entrées et sorties analogiques permettant d'acquérir toutes les informations utiles, aussi bien les valeurs commandées que les valeurs mesurées, relatives au courant et à la tension de chauffage de la source de faisceau de particules, et à l'amplitude d'impulsion en tension de polarisation de la grille.

L'émetteur du rayonnement ionisant est un accélérateur de particules, notamment linéaire, par exemple un accélérateur linéaire d'électrons. Le faisceau d'électrons issu de l'accélérateur peut être utilisé directement comme rayonnement ionisant après avoir traversé une fenêtre (9) qui sépare le vide de l'enceinte de l'accélérateur et l'atmosphère extérieur, ou bien l'accélérateur peut être équipé d'une cible de conversion de la puissance du faisceau d'électrons en rayons X qui constitue alors le rayonnement ionisant utile.

L'accélérateur produit un faisceau de particules d'énergie et de courant souhaités, notamment d'énergie dans une gamme comprise entre 1 MeV et 50 MeV, de préférence la gamme de 3 MeV à 25 MeV. Le courant et l'énergie sont définis par l'opérateur via l'interface homme-machine pour obtenir le rayonnement ionisant souhaité dans la plage choisie précédemment.

Avantageusement le moyen d'accélération (8) est soit à onde hyperfréquence ou soit à induction ou soit électrostatique.

Selon d'autres caractéristiques de l'invention, le moyen d'accélération (8) est particulièrement une cavité accélératrice.

Avantageusement le moyen d'émission de rayonnement (MER) comprend en outre plusieurs moyens d'accélération, notamment une ou plusieurs cavités accélératrices, montées en série et/ou intercalées de moyens de déviation ou de recirculation du faisceau de particules à travers le ou lesdits moyens accélérateurs, chaque moyen accélérateur étant alimenté par au moins une source de puissance laquelle peut être alimentée par au moins un modulateur, ledit modulateur étant lui-même alimenté par une source haute tension HT.

Dans le cas d'un accélérateur linéaire à onde hyperfréquence progressive ou stationnaire, par exemple en bande S à 3 Gigahertz (GHz), un faisceau d'électrons d'énergie ajustable dans une gamme comprise entre 1 MeV et 50 MeV et de courant moyen 100 µA peut être utilisé comme rayonnement ionisant. Tout autre type d'accélérateur de plus haute ou plus basse fréquence, ou même DC, c'est-à-dire électrostatique (c'est-à-dire un accélérateur à tension continue) peut être utilisé tant que la gamme d'énergie et le courant moyen correspondent aux valeurs précédemment indiquées. Par exemple, le faisceau est pulsé à une fréquence (f) ajustable comprise entre 5 Hz et 200 Hz avec une durée d'impulsion (d) ajustable entre 0,05 µs et 4,5 µs et d'amplitude minimale de 100 mA.

Pour les rayons X, il est nécessaire que l'accélérateur soit plus puissant, capable d'accélérer un faisceau primaire d'électrons d'intensité moyenne supérieure à 1 mA. Cet accélérateur peut être un accélérateur linéaire à cavité en cuivre, ou un accélérateur linéaire supraconducteur, ou tout autre type d'accélérateur correspondant aux exigences du résultat attendu objet de la présente invention.

Dans le cas d'un accélérateur linéaire comprenant une cavité en cuivre, il est pulsé à des fréquences (f) supérieures à 200 Hz, avec une durée d'impulsion (d) d'au moins 5 µs et une intensité crête d'au moins 1 A. Il peut être constitué de plusieurs cavités accélératrices en série, chacune alimentée par sa propre source de puissance haute fréquence (HF). Les sources de puissance peuvent être des moyens d'amplification comprenant une chambre à vide et permettant de réaliser des amplifications de moyennes et fortes puissances à bande étroite en hyperfréquences, par exemple les klystrons, et ces moyens d'amplification sont eux même alimentés par des modulateurs classiques ou à état solide.

Pour fournir un faisceau pulsé à fréquence (f) ajustable, une durée d'impulsion (d) ajustable et une amplitude de courant crête ajustable, la source de faisceau de l'accélérateur comprend un canon à électrons DC ou électrostatique (c'est-à-dire un canon à électron à tension continue) de type triode ou de type photocathode déclenché par laser ou de type plasma déclenché par électrode ou par laser. Il peut s'agir par exemple d'un canon à haute tension DC à cathode thermo-ionique à grille. La cathode est portée à un potentiel négatif d'une dizaine à plusieurs dizaines de kilovolts. L'anode reste au potentiel de masse, notamment 0 V (zéro volt). La grille joue alors le rôle de déclencheur en étant portée à un potentiel négatif et inférieur d'une dizaine ou d'une centaine de volts par rapport à la cathode pour ne pas émettre et à un potentiel négatif mais supérieur à celui de la cathode pour émettre. L'amplitude du courant crête émis par la cathode dépend de sa différence de potentiel par rapport à celui de la grille. Des impulsions à fréquence (f) ajustable, à durée (d) ajustable et à amplitude de tension ajustable sont envoyées sur la grille en phase avec les impulsions haute tension (HT) d'alimentation de la cathode pour générer un faisceau pulsé souhaité et injecté pour l'accélération des particules dans les cavités haute fréquence (HF) de l'accélérateur.

Le dispositif selon l'invention présente l'avantage d'offrir à l'opérateur la possibilité de programmer le type de rayonnement ionisant, l'énergie du rayonnement, le débit de dose de rayonnement ionisant absorbée et/ou à délivrer, et la durée de l'irradiation ou la dose de rayonnement ionisant à absorbée et/ou à délivrer.

Il permet de délivrer de manière contrôlée et précise et de manière souhaitée via l'interface homme-machine, de façon intelligente, de fortes doses de rayonnement ionisant d'au moins 0,01 Gy, voire 0,25 Gy avec une précision d'au moins 1 µGy, de préférence 1 nGy, à des énergies dans une gamme comprise entre 1 MeV et 50 MeV, en des instants très brefs typiquement compris entre 0,1 µs et 100 ms, de préférence 100 µs voire 1 ms.

Il permet également, pour d'autres applications, de délivrer de manière contrôlée et précise et de manière souhaitée, via l'interface homme-machine, de forts débits de doses de rayonnement ionisant jusqu'à 250 Gy/s, à des énergies dans des gammes comprises entre 1 MeV et 50 MeV, en des instants très brefs d'au moins 50 ns, à des fins de radiothérapie et/ou radiobiologie.

On voit donc qu'il est possible de réaliser de façon industrielle une machine de radiothérapie et/ou de radiobiologie fiable capable de délivrer une forte dose précise et prescrite de rayonnement ionisant, par exemple de 10 Gy, en des temps très courts, par exemple 100 ms.

Contrairement aux préjugés qui consistaient à croire qu'il est impossible de concevoir une machine de radiothérapie capable de fournir les performances décrites ci-avant, l'invention présentée ici permet de montrer qu'en utilisant la machine décrite dans cette invention, il est possible de résoudre le problème précédemment cité.

## Revendications

1. Dispositif d'irradiation à rayonnement ionisant, notamment pour la radiothérapie et/ou la radiobiologie, comprenant simultanément au moins :

   - un moyen d'émission de rayonnement ionisant (MER) comprenant :

     o au moins une source de particules qui comprend au moins :

       ▪ une cathode et une anode, ou un plasma,
       ▪ un moyen de déclenchement d'émission de particules, notamment une grille ou une électrode ou un laser et
       ▪ un moyen d'accélération d'un faisceau de particules, et

     ◦ un système de contrôle d'impulsion de puissance,

   - un moyen de détection de dose de rayonnement ionisant (MDD) comprenant un détecteur,
   - un moyen de contrôle de dose (MCD), configuré pour contrôler le déclenchement et l'arrêt du moyen d'émission de rayonnement ionisant (MER), comprenant :

     ◦ une électronique de contrôle (EC),
     ◦ un amplificateur et/ou un atténuateur amplifiant et/ou atténuant un signal émis par le détecteur,
     ◦ un intégrateur intégrant pendant la durée de l'émission ledit signal amplifié et/ou atténué et
     ◦ un comparateur comparant continûment ledit signal intégré aux consignes de la dose prescrite (CDA) prédéterminée par un opérateur,

   - un système de contrôle et de commande (SCC) comportant :

     ◦ un système de contrôle fonctionnel (SCF) des différents organes dudit dispositif, comprenant :

       ▪ une interface homme machine (IHM),
       ▪ des outils de visualisation et
       ▪ des moyens de mise en oeuvre configurés pour qu'un opérateur programme, en une seule fois, la nature du rayonnement, le débit de dose absorbé de rayonnement ionisant, une durée de l'émission du rayonnement ionisant, le régime impulsionnel,

   - le dispositif étant **caractérisé en ce que** : lesdits moyens (MER, MDD, MCD) et ledit système (SCC) sont configurés pour délivrer et contrôler des doses de rayonnement ionisant d'au moins 0,01 Gy voire 0,25 Gy avec une précision d'au moins 1 μGy, de préférence 1 nGy, à des énergies comprises entre 1 MeV et 50 MeV, pendant des instants compris entre environ 0,1 μs et 100 ms, de préférence 100 μs ou 1 ms,
   - le système de contrôle d'impulsion de puissance du moyen d'émission de rayonnement ionisant (MER) est configuré pour produire un faisceau de particules, c'est-à-dire un rayonnement ionisant, d'énergie dans une gamme comprise entre 1 MeV et 50 MeV, pulsé à une fréquence (f) comprise entre 5 Hz et 1000 Hz, voire entre 5 Hz et 500 Hz, voire entre 5 Hz et 200 Hz, et de préférence d'environ 100 Hz, avec une durée d'impulsion (d) d'au moins 1 ns, de préférence comprise entre 0,05 μs et 12 μs, de préférence 0,1 μs, et pour délivrer un débit de dose absorbée d'au moins environ 0,01 Gy/s, par exemple compris entre environ 0,01 Gy/s et environ 250 Gy/s, voire 500 Gy/s ou même 1000 Gy/s,
   - l'électronique de contrôle (EC) du moyen de contrôle de dose (MCD) est configurée pour contrôler une dose qui est délivrée pendant au moins 0,1 μs, voire 100 μs, voire 1 ms voire 100 ms et
   - le détecteur est un détecteur ultra-rapide, couplé au moyen de contrôle de dose (MCD), configuré pour détecter une dose de rayonnement ionisant en au moins 0,01 ns et à des débits de dose d'au moins 0,01 Gy/s, par exemple 25 Gy/s, voire 50 Gy/s, voire de préférence 250 Gy/s, voire encore 500 Gy/s ou même encore 1000 Gy/s.

2. Dispositif d'irradiation à rayonnement ionisant selon la revendication 1 **caractérisé en ce que** le moyen d'émission de rayonnement ionisant (MER) est un accélérateur de particules comprenant une source de puissance et un système de contrôle d'impulsion, ledit système de contrôle d'impulsion étant directement relié au moyen de contrôle de dose (MCD) de façon à arrêter automatiquement la source de puissance lorsque la dose absorbée, détectée et mesurée a atteint une valeur prescrite et prédéterminée par l'opérateur via l'interface homme-machine (IHM), ladite interface homme machine (IHM) comprend en outre un poste de commande (PC) et un programme de gestion d'interface de visualisation.

3. Dispositif d'irradiation à rayonnement ionisant selon l'une quelconque des revendications 1 ou 2 **caractérisé en ce que** le moyen d'accélération est soit à onde hyperfréquence, soit à induction, soit électrostatique.

4. Dispositif d'irradiation à rayonnement ionisant selon l'une quelconque des revendications précédentes **caractérisé en ce que** le moyen d'émission de rayonnement (MER) comprend en outre plusieurs moyens d'accélérations montés en série et/ou intercalés de moyens de déviation ou de recirculation du faisceau de particules à travers le ou lesdits moyens accélérations, chacun étant alimenté par au moins une source de puissance.

5. Dispositif d'irradiation à rayonnement ionisant selon l'une quelconque des revendications précédentes **caractérisé en ce que** le détecteur est un semi-conducteur, notamment du diamant sous forme monocristalline ou poly-cristalline, pur ou dopé, compris entre deux électrodes de polarisation aux bornes desquelles est appliquée une tension de quelques volts réglable selon l'épaisseur du détecteur via l'interface homme-machine configuré pour obtenir un temps de réponse très court d'au moins 0,01 ns, à de forts débits de dose d'au moins 0,01 Gy/s.

6. Dispositif d'irradiation à rayonnement ionisant selon l'une quelconque des revendications précédentes **caractérisé en ce que** l'électronique de contrôle (EC) du moyen de contrôle de dose (MCD) comprend des moyens de mesure de courant électrique, notamment d'un signal électrique, produit par l'interaction du rayonnement ionisant avec le détecteur, des moyens de conversion dudit courant électrique en unité de débit de dose absorbée et des moyens d'intégration dudit courant électrique configurés pour mesurer avec précision la dose absorbée cumulée au cours de l'émission du rayonnement ionisant.

7. Dispositif d'irradiation à rayonnement ionisant selon l'une quelconque des revendications précédentes dans lequel un signal électrique est produit par le détecteur, ledit signal électrique est mesuré et intégré par un électromètre au cours de l'émission du rayonnement ionisant, la valeur intégrée dudit signal électrique est directement comparée à la valeur de la dose prescrite et prédéterminée par l'opérateur via l'interface homme-machine de façon à ce que, dès que la valeur intégrée est supérieure ou égale à la valeur prescrite et prédéterminée, le signal du moyen de déclenchement d'émission de particules est interrompu pour arrêter instantanément l'émission du rayonnement ionisant, et de préférence la source haute tension, en au moins 1 ns.

8. Dispositif d'irradiation à rayonnement ionisant selon l'une quelconque des revendications précédentes dans lequel le détecteur comprend un ou plusieurs quadrants ou secteurs ou voxels produisant chacun un signal de détection (SDA) et configurés pour permette de déduire de leurs signaux de détection (SDA) des informations caractérisant la position, la forme et/ou l'énergie du faisceau de rayonnement ionisant qui les traverse d'une part, et de façon à contrôler et réguler le faisceau de rayonnement ionisant, notamment sa position, sa forme et/ou son énergie d'autre part.

## Patentansprüche

1. Vorrichtung zum Bestrahlen mit einer ionisierenden Strahlung, insbesondere für die Radiotherapie und/oder Radiobiologie, die zugleich zumindest enthält:

    - eine Einrichtung zum Emittieren von ionisierender Strahlung (MER), umfassend:

      ◦ zumindest eine Teilchenquelle, die zumindest enthält:

        ■ eine Kathode und eine Anode oder ein Plasma,
        ■ eine Auslöseeinrichtung zum Auslösen der Emission von Teilchen, insbesondere ein Gitter oder eine Elektrode oder einen Laser, und
        ■ eine Beschleunigungseinrichtung zum Beschleunigen eines Teilchenstrahls, und

      ◦ ein Leistungsimpulssteuersystem,

    - eine Erfassungseinrichtung zum Erfassen ionisierender Strahlungsdosen (MDD), die einen Detektor enthält,
    - eine Dosissteuereinrichtung (MCD), die dazu ausgelegt ist, um das Auslösen und Anhalten der Einrichtung zum Emittieren von ionisierender Strahlung (MER) zu steuern, umfassend:

    ◦ eine Steuerelektronik (EC),
    ◦ einen Verstärker und/oder einen Dämpfer, der ein von dem Detektor emittiertes Signal verstärkt und/oder abdämpft,
    ◦ einen Integrator, der für die Dauer der Emission das verstärkte und/oder abgedämpfte Signal integriert, und
    ◦ einen Komparator, der das integrierte Signal kontinuierlich mit den Sollwerten der vorgeschriebenen, durch einen Bediener vorbestimmten Dosis (CDA) vergleicht,

- ein Steuerungs- und Kontrollsystem (SCC), umfassend:

    ◦ ein Funktionssteuersystem (SCF) für die verschiedenen Organe der Vorrichtung, umfassend:

        ▪ eine Mensch-Maschine-Schnittstelle (IHM),
        ▪ Visualisierungsmittel und
        ▪ Implementierungsmittel, die dazu ausgelegt sind, dass ein Bediener die Art der Strahlung, die Rate absorbierter Dosis der ionisierenden Strahlung, eine Emissionsdauer der ionisierenden Strahlung und den Pulsbetrieb auf einmal programmiert,

- wobei die Vorrichtung **dadurch gekennzeichnet ist, dass** die Einrichtungen (MER, MDD, MCD) und das System (SCC) dazu ausgelegt sind, ionisierende Strahlungsdosen von zumindest 0,01 Gy bzw. 0,25 Gy mit einer Genauigkeit von zumindest 1 $\mu$Gy, vorzugsweise 1 nGy, mit Energien zwischen 1 MeV und 50 MeV für Zeitpunkte zwischen etwa 0,1 $\mu$s und 100 ms, vorzugsweise 100 $\mu$s oder 1 ms, bereitzustellen und zu steuern,
- das Leistungsimpulssteuersystem der Einrichtung zum Emittieren von ionisierender Strahlung (MER) dazu ausgelegt ist, einen Teilchenstrahl, d. h. eine ionisierende Strahlung, mit einer Energie im Bereich zwischen 1 MeV und 50 MeV zu erzeugen, gepulst mit einer Frequenz (f) zwischen 5 Hz und 1000 Hz bzw. zwischen 5 Hz und 500 Hz bzw. zwischen 5 Hz und 200 Hz und vorzugsweise von etwa 100 Hz mit einer Pulsdauer (d) von mindestens 1 ns, vorzugsweise zwischen 0,05 $\mu$s und 12 $\mu$s, vorzugsweise von 0,1 $\mu$s zu erzeugen und eine Rate absorbierter Dosis von zumindest etwa 0,01 Gy/s, beispielsweise zwischen etwa 0,01 Gy/s und etwa 250 Gy/s bzw. 500 Gy/s oder sogar 1000 Gy/s abzugeben,

- die Steuerelektronik (EC) der Dosissteuereinrichtung (MCD) dazu ausgelegt ist, eine Dosis zu steuern, die für mindestens 0,1 $\mu$s bzw. 100 $\mu$s bzw. 1 ms bzw. 100 ms abgegeben wird, und
- der Detektor ein ultraschneller Detektor ist, der mit der Dosissteuereinrichtung (MCD) gekoppelt und dazu ausgelegt ist, eine Dosis ionisierender Strahlung in wenigstens 0,01 ns bei Dosisraten von zumindest 0,01 Gy/s, beispielsweise 25 Gy/s bzw. 50 Gy/s bzw. vorzugsweise 250 Gy/s bzw. 500 Gy/s oder sogar 1000 Gy/s zu detektieren.

2.   Vorrichtung zum Bestrahlen mit einer ionisierenden Strahlung nach Anspruch 1, **dadurch gekennzeichnet, dass** die Einrichtung zum Emittieren von ionisierender Strahlung (MER) ein Teilchenbeschleuniger mit einer Leistungsquelle und einem Impulssteuersystem ist, wobei das Impulssteuersystem direkt mit der Dosissteuereinrichtung (MCD) verbunden ist, so dass automatisch die Leistungsquelle abgeschaltet wird, wenn die absorbierte, detektierte und gemessene Dosis einen vorgeschriebenen und vom Bediener über die Mensch-Maschine-Schnittstelle (IHM) vorbestimmten Wert erreicht, wobei die Mensch-Maschine-Schnittstelle (IHM) ferner eine Steuerstation (PC) und ein Verwaltungsprogramm für die Visualisierungsschnittstelle enthält.

3.   Vorrichtung zum Bestrahlen mit einer ionisierenden Strahlung nach einem der Ansprüche 1 oder 2, **dadurch gekennzeichnet, dass** die Beschleunigungseinrichtung entweder vom Typ mit Höchstfrequenzwelle, Induktion oder vom elektrostatischen Typ ist.

4.   Vorrichtung zum Bestrahlen mit einer ionisierenden Strahlung nach einem der vorherigen Ansprüche, **dadurch gekennzeichnet, dass** die Strahlungsemissionseinrichtung (MER) ferner mehrere Beschleunigungseinrichtungen enthält, die mit Einrichtungen zum Umlenken oder Rückführen des Teilchenstrahls durch die Beschleunigungseinrichtung bzw. Beschleunigungseinrichtungen in Reihe geschaltet und/oder zwischengeschaltet sind, wobei sie jeweils von zumindest einer Leistungsquelle gespeist werden.

5.   Vorrichtung zum Bestrahlen mit einer ionisierenden Strahlung nach einem der vorherigen Ansprüche, **dadurch gekennzeichnet, dass** der Detektor ein Halbleiter ist, insbesondere aus Diamant in monokristalliner oder polykristalliner Form, rein oder dotiert, der zwischen zwei Vorspannungselektroden enthalten ist, an deren Anschlüssen eine Spannung von einigen Volt angelegt ist, die je nach Dicke des Detektors über die Mensch-Maschine-Schnittstelle einstellbar ist, und dazu ausgelegt ist, eine sehr kurze Reaktionszeit von mindestens 0,01 ns mit hohen Dosisraten von mindestens 0,01 Gy/s zu erhalten.

6. Vorrichtung zum Bestrahlen mit einer ionisierenden Strahlung nach einem der vorherigen Ansprüche, **dadurch gekennzeichnet, dass** die Steuerelektronik (EC) der Dosissteuereinrichtung (MCD) Einrichtungen zum Messen des elektrischen Stroms, insbesondere eines elektrischen Signals, enthält, das durch Wechselwirkung der ionisierenden Strahlung mit dem Detektor erzeugt wird, sowie Einrichtungen zum Umwandeln des elektrischen Stroms in Rateneinheiten absorbierter Dosis und Einrichtungen zum Integrieren des elektrischen Stroms, die dazu ausgelegt sind, die im Laufe der Emission der ionisierenden Strahlung kumulierte absorbierte Dosis präzise zu messen.

7. Vorrichtung zum Bestrahlen mit einer ionisierenden Strahlung nach einem der vorherigen Ansprüche, bei welcher ein elektrisches Signal von dem Detektor erzeugt wird, wobei das elektrische Signal über ein Elektrometer im Laufe der Emission der ionisierenden Strahlung gemessen und integriert wird, wobei der integrierte Wert des elektrischen Signals direkt mit dem Wert der vorgeschriebenen und durch den Bediener über die Mensch-Maschine-Schnittstelle vorbestimmten Dosis verglichen wird, so dass dann, wenn der integrierte Wert größer oder gleich dem vorgeschriebenen, vorbestimmten Wert ist, das Signal der Auslöseeinrichtung zum Auslösen der Teilchenemission unterbrochen wird, um augenblicklich die Emission der ionisierenden Strahlung, vorzugsweise die Hochspannungsquelle, innerhalb von mindestens 1 ns anzuhalten.

8. Vorrichtung zum Bestrahlen mit einer ionisierenden Strahlung nach einem der vorherigen Ansprüche, bei welcher der Detektor einen oder mehrere Quadranten oder Sektoren oder Voxel enthält, die jeweils ein Erfassungssignal (SDA) erzeugen und so ausgelegt sind, dass sie ermöglichen, einerseits von ihren Erfassungssignalen (SDA) Informationen abzuleiten, die die Position, die Form und/oder die Energie der ionisierenden Strahlung charakterisieren, die durch sie hindurchtritt, und andererseits die ionisierende Strahlung zu steuern und zu regeln, insbesondere ihre Position, ihre Form und/oder ihre Energie.

## Claims

1. An irradiation device using ionizing radiation, in particular for radiotherapy and/or radiobiology, simultaneously comprising at least:

- a means for emitting ionizing radiation (MER) comprising:

  ○ at least one particle source which comprises at least:

    ■ a cathode and an anode, or a plasma,
    ■ a means for triggering particle emission, in particular a grid or an electrode or a laser and
    ■ a means for accelerating a particle beam, and

  ○ a system for power pulse control,

- an ionizing radiation dose detection means (DDM) comprising a detector,
- a dose control means (DCM), configured to control the triggering and the stopping of the means for emitting ionizing radiation (MER), comprising:

  ○ control electronics (CE),
  ○ an amplifier and/or an attenuator amplifying and/or attenuating a signal emitted by the detector,
  ○ an integrator integrating, during the time of the emission, said amplified and/or attenuated signal and
  ○ a comparator continuously comparing said integrated signal to the settings for the specified dose (SAD) predetermined by an operator,

- a control and actuating system (CAS) comprising:

  ○ a system for functional control (SFC) of the various members of said device, comprising:

    ■ a man-machine interface (MMI),
    ■ viewing tools and
    ■ implementation means configured in order for an operator to program, at a single time, the nature of the radiation, the absorbed dose rate of ionizing radiation, a duration for the emission of the ionizing radiation, the pulse settings,

- the device being **characterized in that**: said means (MER, DDM, DCM) and said system (CAS), are configured for delivering and controlling doses of ionizing radiation of at least 0.01 Gy or even 0.25 Gy with an accuracy of at least 1 $\mu$Gy, preferably 1 nGy, at energies comprised between 1 MeV and 50 MeV, for times comprised between approximately 0,1 $\mu$s and 100 ms, preferably 100 $\mu$s or 1 ms,

- the power pulse control system of the means for emitting ionizing radiation (MER) is configured for producing a particle beam, that is to say ionizing radiation, of energy in a range comprised between 1 MeV and 50 MeV, pulsed at a frequency (f) comprised between 5 Hz and 1000 Hz, or even between 5 Hz and 500 Hz, or even between 5 Hz and 200 Hz, and preferably approximately 100 Hz, with a pulse duration (d) of at least 1 ns, preferably comprised between 0.05 $\mu$s and 12 $\mu$s, preferably 0.1 $\mu$s, and for delivering an absorbed dose rate of at least approximately 0.01 Gy/s, for example comprised between approximately 0.01 Gy/s and approximately 250 Gy/s, or even 500 Gy/s or even 1000 Gy/s,

- the control electronics (CE) of the dose control means (DCM) is configured for controlling a dose which is delivered for at least 0.1 $\mu$s, or even 100 $\mu$s, or even 1 ms, or even 100 ms and

- the detector is an ultra-fast detector, coupled to a dose control means (DCM), configured to detect a dose of ionizing radiation in at least 0.01 ns and at dose rates of at least 0.01 Gy/s, for example 25 Gy/s, or even 50 Gy/s, or even preferably 250 Gy/s, or even 500 Gy/s or even 1000 Gy/s.

2. An irradiation device using ionizing radiation according to claim 1, **characterized in that** the means for emitting ionizing radiation (MER) is a particle accelerator comprising a power source and a pulse control system, said pulse control system being directly linked to the dose control means (DCM) so as to automatically stop the power source when the absorbed dose, detected and measured, has attained a value specified and predetermined by the operator via the man-machine interface (MMI), said man machine interface (MMI) further comprises a command station (CS) and a viewing interface management program.

3. An irradiation device using ionizing radiation according to any one of claims 1 or 2, **characterized in that** the acceleration means is either microwave frequency or induction or electrostatic based.

4. An irradiation device using ionizing radiation according to any one of the preceding claims, **characterized in that** the means for emitting radiation (MER) further comprises several acceleration means mounted in series and/or interleaved with means for deviation or recirculation of the particle beam through the one or more acceleration means, each being supplied by at least one power source.

5. An irradiation device using ionizing radiation according to any one of the preceding claims, **character-**

**ized in that** the detector is a semiconductor, in particular diamond in monocrystalline or polycrystalline form, pure or doped, comprised between two polarization electrodes at the terminals of which is applied a potential of several volts adjustable according to the thickness of the detector via the man-machine interface, and is configured to obtain a very short response time of at least 0.01 ns, at high dose rates of at least 0.01 Gy/s.

6. An irradiation device using ionizing radiation according to any one of the preceding claims, **characterized in that** the control electronics (CE) of the dose control means (DCM) comprises means for measuring electric current, in particular of an electrical signal, produced by the interaction of the ionizing radiation with the detector, means for converting said electric current into absorbed dose rate units and means for integrating said electric current that are configured to accurately measure the absorbed dose accumulated during the emission of the ionizing radiation.

7. An irradiation device using ionizing radiation according to any one of the preceding claims, wherein an electrical signal is produced by the detector, said electrical signal is measured and integrated by an electrometer during the emission of the ionizing radiation, the integrated value of said electrical signal is directly compared to the value of the dose specified and predetermined by the operator via the man-machine interface such that, as soon as the integrated value is greater than or equal to the specified and predetermined value, the signal of the means for triggering particle emission is interrupted to instantaneously stop the emission of the ionizing radiation, and preferably the high voltage source, in at least 1 ns.

8. An irradiation device using ionizing radiation according to any one of the preceding claims, wherein the detector comprises one or more quadrants or sectors or voxels each producing a detection signal (ADS) and configured to enable information to be deduced, from their detection signals (ADS), characterizing the position, the shape and/or the energy of the ionizing radiation beam which passes through them, and furthermore so as to control and regulate the ionizing radiation beam, in particular its position, its shape and/or its energy.

Fig. 1

Fig. 2

Fig. 3

Fig. 4

EP 3 071 292 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2012085507 A
- WO 2007017177 A
- US 2010329413 A
- US 6445766 B
- US 7567647 B
- US 2008144772 A